# EUROPEAN PATENT SPECIFICATION

(11) **EP 2 805 714 B1**
(45) Date of publication and mention of the grant of the patent: **25.10.2017**
(21) Application number: 14464003.4
(22) Date of filing: 20.02.2014
(51) Int. Cl.: A61K 9/20, A61K 31/505

(54) **STABLE PHARMACEUTICAL COMPOSITION COMPRISING AMORPHOUS ROSUVASTATIN CALCIUM**
STABILE PHARMAZEUTISCHE ZUSAMMENSETZUNG MIT AMORPHEM ROSUVASTATINCALCIUM
COMPOSITION PHARMACEUTIQUE STABLE COMPRENANT DE LA ROSUVASTATINE CALCIQUE AMORPHE

(30) Priority: 25.04.2013 RO 201300322
(43) Date of publication of application: 26.11.2014
(73) Proprietor: Antibiotice S.A., 707410 Iasi (RO)
(72) Inventor: POTUR, Roxana-Georgiana, Iasi (RO); MOISUC, Lacramioara Stefania, Iasi (RO); Saraficeanu, Nicoleta, Iasi (RO); BOITA, Tudor, Iasi (RO); MACOVEI, Lenuta, Iasi (RO); TELISÇA, Amalia-Diana, Iasi (RO)
(74) Representative: Bösl, Raphael Konrad

(56) References cited:
- EP-A1- 0 547 000
- WO-A1-2006/134604
- WO-A1-2009/156796
- WO-A2-2009/024889
- WO-A2-2011/018185

## Description

### Technical Aspect

The present invention relates to a novel stable pharmaceutical composition of amorphous rosuvastatin calcium, bioequivalent to the innovator product, that contains an optimal ratio of insoluble stabilizing salt and soluble filler, more specifically calcium carbonate and lactose monohydrate in the range between 1:3 to 1:14 by weight. The stability of the drug substance is also provided by the coating based on polyvinyl alcohol.

### Background of the Invention

Rosuvastatin, a hydroxymethylglutaryl coenzyme A (HMG-CoA) reductase inhibitor (statin), is a lipid regulating drug with actions on plasma lipids similar to those of simvastatin. Its chemical name is bis [(E)- 7-[4-(4-fluorophenyl)-6-isopropyl-2-[methyl(methylsulfonyl)amino]pyrimidin-5-yl]-(3R,5S)-dihydroxyhept-6-enoic acid] calcium salt and it is represented in Formula I.

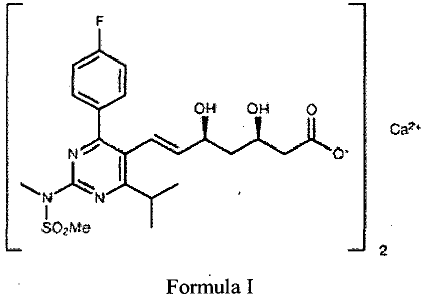

Rosuvastatin is used to reduce LDL-cholesterol, apolipoprotein B, and triglycerides of hyperlipidaemias, including primary hypercholesterolaemia (type IIa), mixed dyslipidaemia (type IIb), and hypertriglyceridaemia (type IV), as well as in patients with homozygous familial hypercholesterolaemia. It is also used to reduce the progression of atherosclerosis.

Rosuvastatin is administered orally as the calcium salt, although doses are expressed in terms of the base: 10.4 mg of rosuvastatin calcium is equivalent to about 10 mg of base. The originator product is marketed by AstraZeneca under the brand names Crestor®, Rosuvast®, Rovartal®, Cresadex®, Rosumed® and Sinlip®.

It is known that HMG-CoA reductase inhibitors such as rosuvastatin calcium are unstable under conditions of acidity, oxygen, light and humidity. Rosuvastatin calcium suffers degradation to [(E)-7-[4-(4-fluorophenyl)-6-isopropyl-2-[methyl(methylsulfonyl)amino]pyrimidin-5-yl]- -(3R,5S) 3,5-dihydroxyhept-6-enoic acid-3.6] lactone (reffered to as "lactone"), an oxidation product and when exposed to light, it undergoes to some other isomers. For this reasons it is very difficult to formulate a pharmaceutical product and to provide a composition which is stable throughout the shelflife.

The low stability of this compound functioned as a reason for the use in the prior art of crystalline rosuvastatin calcium, which can be more stable than the amorphous compound. It is also well known that an amorphous drug substance can be more hygroscopic than a crystalline one.

In patent EP 1144389 B1, 23.12.1999, AstraZeneca disclosed the polymorphic form A of rosuvastatin calcium and its pharmaceutical compositions.

In publication EP 1663989 B1, 08.09.2009, AstraZeneca also claims the polymorphic forms B, B1 and their pharmaceutical compositions.

In patent application EP 1912952 A1, 22.06.2006, Lek Pharmaceuticals discloses a process for preparing amorphous rosuvastatin calcium free of impurities, with increased stability.

Stable pharmaceutical formulations of 7-substituted -3,5-didihydroxy-6-heptenoic acid salts (HMG CoA reductase inhibitor), are also disclosed in EP 0547000 B1, 08.12.1992, by Novartis AG and require the presence of an alkaline medium capable of imparting a pH of at least 8 to an aqueous solution or dispersion of the composition.

The use of alkalizing agents (such as calcium carbonate) has the following yet important drawback: they are sparingly soluble in water and react slowly with gastric HCl. All antacids increase intragastric pH and may decrease the bioavailability of drugs that require gastric acidity for their absorbtion. The main advantage consists in the lowering of the hygroscopic manifestations of the active ingredient in the pharmaceutical form.

In EP-1223918 B1, 04.08.2000, AstraZeneca AB disclosed a pharmaceutical composition comprising rosuvastatin, wherein the stability of rosuvastatin is improved, with an inorganic salt in which the cation is multivalent, preferably tribasic calcium phosphate.

Being a poor water soluble, low bioavailability compound, belonging to BCS class II, the rate of absorbtion and bioavailability of rosuvastatin calcium can be influenced directly by the use of alkalizing agents and may be controlled by the type of drug substance used (crystalline, non-crystalline) and its rate of dissolution in the gastrointestinal tract. There are many known methods of enhancing the dissolution charactheristics of slightly water-soluble drugs, such as .the use of amorphous drug substances and combination with soluble fillers, superdisintegrants and solubilizing agents. Higher solubility and faster dissolution rates may lead to significantly better oral bioavailability.

From a drug dissolution point of view, formulators need to consider the solubility of both the filler and the drug. It was demonstrated that dissolution of poorly soluble drugs improves greatly with the concentration of soluble fillers in the formulation.

In prior art, there are many examples regarding the use of a soluble filler , such as lactose monohydrate, in solid formulations of rosuvastatin calcium. In EP 2464344 A2, 13.08.2009, Synthon B.V. claims an embodiment with rosuvastatin calcium, dibasic calcium phosphate, a binder such as microcrystalline cellulose and a water-soluble diluent such as lactose (including lactose monohydrate).

The patent WO 2008/035128 A1 applied by Gedeon Richter (Hungary) is related to a pharmaceutical composition that comprises rosuvastatin calcium as the active ingredient and magnesium hydroxide, calcium acetate, calcium gluconate, calcium glycerophsphate or aluminum hydroxide as stabilizing excipients and one or more acceptable pharmaceutical excipients such as lactose, microcrystalline cellulose, PVP, crospovidone and magnesium stearate.

From all the information stated in previous literature, the sensitivity of the active substance, its stability throughout the shelf-life, its bioavailability in the pharmaceutical form and the bioequivalence of the formulation with the originator product depend directly on the form of the active substance (crystalline/non-crystalline) and on the pharmaceutical excipients used in the formulation.

An amorphous form of the active substance may be formulated to enhance the oral bioavailability of a poorly soluble drug, such as rosuvastatin calcium. The dissolution enhancement can be translated into a marked improvement in both AUC and Cmax. Despite physical and chemical instability of amorphous material that has prevented widespread applications of amorphous form for bioavailability enhancement, the economical criterion is still a strong one: the amorphous forms of are less expensive to manufacture.

As a consequence, there is a imperious necessity in the field of pharmaceutical industry to develop robust, reproducible pharmaceutical formulations with amorphous rosuvastatin calcium, stabilized with an insoluble alkalizing agent (that prevents hygroscopicity, such as calcium carbonate), combined with a soluble filler (such as lactose monohydrate), in order to provide bioavailability to the active substance, stability over shelf-life, and bioequivalence with the originator product.

### Description of the invention

The present invention is based on the necessity of providing a novel pharmaceutical composition of rosuvastatin calcium amorphous with enhanced solubility and stability through shelf-life, bioequivalent with the originator product.

Stabilizers can be selected from the group of antioxidants, chelating agents, alkalizing agents and photo protectors. The alkalizing agents may be chosen from the group of alkali metal salts like sodium carbonate, sodium silicate and earth alkali metal salts like calcium carbonate, calcium phosphate, calcium acetate, calcium gluconate. In this present invention the selected stabilizer is calcium carbonate. The preferred ratio of rosuvastatin calcium to calcium carbonate is in the range of 0.5:1 to 2:1 by weight.

Because of the use of an insoluble alkalizing agent in the proximity of the poorly soluble drug, in order to increase the water uptake of the tablet, lactose monohydrate and a superdisintegrant were used.

The main embodiment of the invention consists in finding the suitable ratio between the insoluble stabilizer and the soluble filler, that ensures the desired formulation characteristics (solubility, stability during shelf-life and bioequivalence). The ratio of calcium carbonate to lactose monohydrate is in the range of 1:3 to 1:14, by weight.

Suitable disintegrants may include but are not limited to crospovidone, croscarmellose sodium, polyvinylpyrrolidone. In this present invention the preferred superdisintegrant is crospovione, in ratio of 1 - 5%

Suitable binders may include but not limited to microcrystalline cellulose, sugars, methylcellulose, hydroxypropyl methylcellulose, hydroxyethylcellulose. In this present invention the most preferred binder is microcrystalline cellulose, in a proportion of 15 - 35%.

The pharmaceutically acceptable lubricants can be selected from the group of metallic stearates, such as magnesium stearate, calcium stearate, fatty acid esters, such as sodium stearyl fumarate, fatty acids, such as stearic acid. The lubricant present in this formulation is magnesium stearate, preferably in a range of 0.1 -10% by weight, more preferably in a range of 0.25-5% by weight.

The pharmaceutical compositions according to the present invention may further comprise one or more pharmaceutically accepted colorants in the coating, such as aluminium lakes, titanium dioxide. The coating preferred for this invention is water based, the film former consisting of polyvinyl alcohol; it has titanium dioxide as UV absorbant and it is free of iron oxides. It provides low moisture uptake rates, excellent elasticity and adhesion and photostability.

The pharmaceutical composition of the present invention can be prepared, using a manufacture process based on direct compression, ensuring the stability of amorphous rosuvastatin, through the absence of granulation solvents. One of the main embodiments of this invention is directed to tablet coating, that can be applied preferable by spray coating the water based film.

The pharmaceutical compositions of this present invention may be formulated for administration to humans, for treating hyperlipoproteinemia, hypercholesterolemia and atherosclerosis.

The invention is further defined by reference to the following examples.

### Example 1: Pharmaceutical composition of amorphous rosuvastatin calcium

| **Ingredients** | **Quantity mg/tablet** |
|---|---|
| Rosuvastatin calcium amorphous* | 5.2 |
| Calcium carbonate | 10.4 |
| Lactose monohydrate | 104 |
| Crospovidone | 7.5 |
| Microcrystalline cellulose | 37.6 |
| Magnesium stearate | 1.5 |
| ***Coating*** | 5 |
| Polyvinyl alcohol partially hydrolyzed | |
| Titanium dioxide | |

| | |
|---|---|
| *equivalent to 5 mg of rosuvastatin | |

### Example 2: Pharmaceutical composition of amorphous rosuvastatin calcium

| **Ingredients** | **Quantity mg/tablet** |
|---|---|
| Rosuvastatin calcium amorphous* | 10.4 |
| Calcium carbonate | 10.4 |
| Lactose monohydrate | 104 |
| Crospovidone | 7.5 |
| Microcrystalline cellulose | 16.2 |
| Magnesium stearate | 1.5 |
| ***Coating*** | 5 |
| Polyvinyl alcohol partially hydrolyzed | |
| Titanium dioxide | |

| | |
|---|---|
| *equivalent to 10 mg of rosuvastatin | |

### Example 3: Pharmaceutical composition of amorphous rosuvastatin calcium

| **Ingredients** | **Quantity mg/tablet** |
|---|---|
| Rosuvastatin calcium amorphous* | 20.8 |
| Calcium carbonate | 20.8 |
| Lactose monohydrate | 208 |
| Crospovidone | 15 |
| Microcrystalline cellulose | 32.4 |
| Magnesium stearate | 3 |
| ***Coating*** | 10 |
| Polyvinyl alcohol partially hydrolyzed | |
| Titanium dioxide | |

| | |
|---|---|
| *equivalent to 20 mg of rosuvastatin | |

### Example 4: Pharmaceutical composition of amorphous rosuvastatin calcium

| **Ingredients** | **Quantity mg/tablet** |
|---|---|
| Rosuvastatin calcium amorphous* | 41.6 |
| Calcium carbonate | 20.8 |
| Lactose monohydrate | 208 |
| Crospovidone | 15 |
| Microcrystalline cellulose | 11.6 |
| Magnesium stearate | 3 |
| ***Coating*** | 10 |
| Polyvinyl alcohol partially hydrolyzed | |
| Titanium dioxide | |

| | |
|---|---|
| *equivalent to 40 mg of rosuvastatin | |

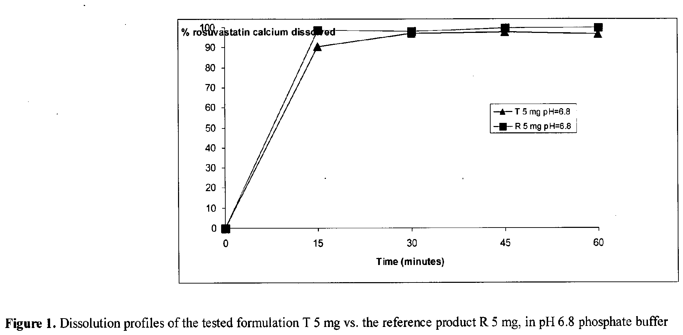

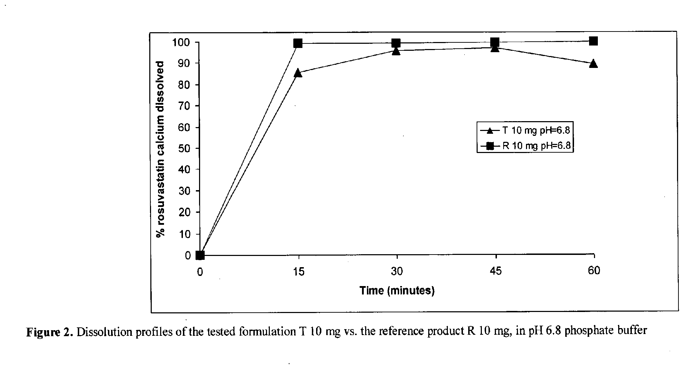

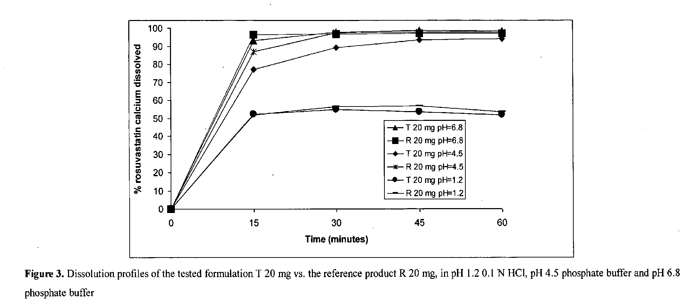

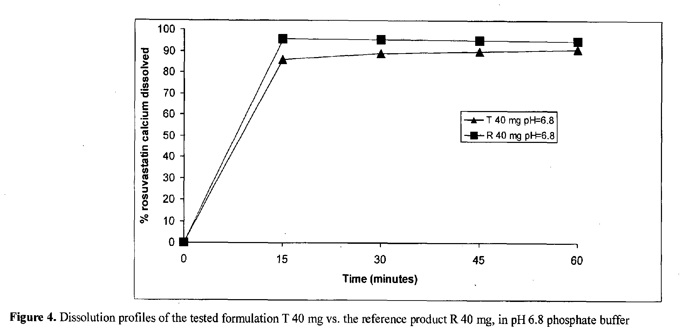

We characterized the drug release from the tested formulation (T) and from the reference product (R) using USP apparatus II at 75 rpm in 900 ml of various media including pH 1.2 0.1 N HCl, pH 4.5 phosphate buffer and pH 6.8 phosphate buffer as shown in Figures 1, 2, 3 and 4.

The similarity factor was used to compare the tested formulation and the standard products' dissolution profiles (Table 1).

**Table 1. Values of the similarity factor for dissolution in pH 1.2, 4.5 and 6.8 media**

| pH media | f₂ value (%) | |
|---|---|---|
| 6.8 | f₂ T vs. R 5 mg | 66.15 |
| 6.8 | f₂ T vs. R 10 mg | 52.34 |
| 6.8 | f₂ T vs. R 20 mg | 81.58 |
| 6.8 | f₂ T vs. R 40 mg | 58.16 |
| 4.5 | f₂ T vs. R 20 mg | 57.62 |
| 1.2 | f₂ T vs. R 20 mg | 82.70 |

These results demonstrate a very good similarity between the in vitro behavior of the formulation related to this invention and the innovator products' (f2 ≥ 50%) in all three dissolution media.

We performed stability tests on these tablet compositions, according to the method described in ICH guidelines. The tablets were packed in opaque OPA/Al/PVC-Al blisters with very low oxygen and humidity transmission rates. Using a HPLC in house method, we measured the level of lactone and total impurities formed after a period of storage of 12 months at 25°C/60%RH (normal conditions, A1) and of 6 months at 40°C/60%RH (accelerated conditions, A2). The results were compared with those obtained for the tablets at the moment of release (TO) and those obtained for the rosuvastatin calcium amorphous active substance (Table 2).

**Table 2. Related substances values for the tested formulations**

| Related substances | RC amorphous | Example 1 (5 mg tablet) | | | Example 2 (10 mg tablet) | | | Example 3 (20 mg tablet) | | | Example 4 (40 mg tablet) | | |
|---|---|---|---|---|---|---|---|---|---|---|---|---|---|
| | | T0 | A1 | A2 | T0 | A1 | A2 | T0 | A1 | A2 | T0 | A1 | A2 |
| Lactone, % | 0.010 | 0.016 | 0.066 | 0.177 | 0.018 | 0.085 | 0.184 | 0.021 | 0.093 | 0.184 | 0.024 | 0.095 | 0.195 |
| Total impurities, % | 0.150 | 0.072 | 0.255 | 0.340 | 0.084 | 0.273 | 0.347 | 0.074 | 0.279 | 0.360 | 0.085 | 0.298 | 0.370 |

In the tablet compositions of the present invention, the maximum level of lactone is found to develop after 6 months of storage at 40°C/60%RH (accelerated conditions), to a value of 0.195 % for the 40 mg strength.
In case of storage for 12 months at 25°C/60%RH (normal conditions), the level of lactone reached a maximum value of 0.095% for the 40 mg strength.

When compared to the value of the lactone obtained for the active substance (0.010 %) and with that obtained for the tablet at the moment of release (0.024%), we observed its slight increase from raw material to the final product (at release and during the period of 1 year of storage in normal conditions).

Extrapolation of 12 months long term data to 24 months shelf life is appropriate, because no significant change has occurred through 6 months at accelerated conditions. The long term data indicates that the formulation related to this invention indicates a stable product, intended for storage at controlled room temperature.

This way, we have demonstrated the stability of the amorphous rosuvastatin calcium in the final pharmaceutical formulation. The alkaline stabilization agent, the PVA-based coating and the low oxygen and humidity transmission rate primary package functioned altogether in a quality end product.

In order to demonstrate the bioequivalence of the formulation with the innovator product, Crestor®, AstraZeneca, .a single dose, fasting state, 2-way crossover study was performed. The study objective was that of comparing the rate and extent of absorption of a single oral dose of 20 mg rosuvastatin in 28 healthy male and female Caucasian subjects under fasting conditions and to evaluate the safety and tolerability of the tested medicinal products.

The results of the trial are illustrated in Tables 3 and 4.

**Table 3. Summary of pharmacokinetic results for rosuvastatin**

| Parameters | Test | | Reference | |
|---|---|---|---|---|
| | Mean ± SD | CV% | Mean ± SD | CV% |
| Cmax (ng/ml) | 8.44 ± 5.56 | 65.87 | 9.16 ± 8.49 | 92.66 |
| AUClast (ng*h /ml) | 74.23 ± 41.59 | 56.03 | 73.60 ± 47.01 | 63.88 |
| AUCtotal (ng*h/ml) | 78.73 ± 42.78 | 54.34 | 78.75 ± 49.59 | 62.96 |
| AUCe (%) | 6.57 ± 3.96 | 60.33 | 7.23 ± 4.44 | 61.39 |
| Tmax (h) | 3.46 ± 1.51 | 43.67 | 3.50 ± 1.63 | 46.71 |
| thalf (h) | 20.20 ± 10.07 | 49.82 | 21.05 ± 10.79 | 51.27 |
| Kel(h-1) | 0.04 ± 0.02 | 44.03 | 0.04 ± 0.02 | 49.79 |

**Table 4. Test vs. Reference Product - Log-transformed Parameters for rosuvastatin**

| | Cₘₐₓ | AUC₀₋ₜ | AUC_{0-∞} |
|---|---|---|---|
| Point estimates (%) | 106 | 105 | 105 |
| 90 % CI | 91.065 - 123.42 | 93.012 - 118.84 | 93.012 - 118. 184 |

No major side effects and no relevant differences in safety profiles were noted between the tested formulation and the innovator product, administered in a single dose of 20 mg rosuvastatin under fasting conditions.

## Claims

1. A pharmaceutical composition of a compressed tablet, which contains amorphous rosuvastatin calcium, calcium carbonate as a stabilizing agent and lactose monohydrate as a soluble filler, coated with a polyvinyl alcohol based film, free of iron oxides, wherein calcium carbonate and lactose monohydrate are in a ratio from 1:3 to 1:14.

2. The pharmaceutical composition according to claim 1, wherein rosuvastatin calcium is present in the core in a proportion of 2 - 20%, particularly 3.467 - 13.867%.

3. The pharmaceutical composition according to claim 1-2, wherein lactose monohydrate is present in the core in a proportion of 30 - 60%.

4. The pharmaceutical composition according to claim 1-3, wherein microcrystalline cellulose is present in the core in a proportion of 15 - 35%.

5. The pharmaceutical composition to claim 1-4, wherein crospovidone is present in the core in a proportion of 1 - 5%.

6. The pharmaceutical composition according to claim 1-5, wherein magnesium stearate is present in the core in a proportion of 0.25 - 5%.

7. A process for manufacturing a stable pharmaceutical tablet comprising rosuvastatin, according according to claim 1-6, consisting of:
a. compressing the powder blend
b. coating the compressed core with a film free of iron oxides, to a weight gain of 2 -10%, particularly, 3 - 4%.

8. The pharmaceutical composition according to claims 1-7, wherein the said core comprises 1-40 mg rosuvastatin, particularly 5, 10, 20, 40 mg.

## Patentansprüche

1. Pharmazeutische Zusammensetzung einer gepressten Tablette, die amorphes Rosuvastatin-Calcium, Calciumcarbonat als Stabilisierungsmittel und Lactosemonohydrat als löslichen Füllstoff enthält, mit einem polyvinylalkoholbasierten Film beschichtet ist, frei von Eisenoxiden ist, wobei Calciumcarbonat und Lactosemonohydrat in einem Verhältnis von 1:3 bis 1:14 sind.

2. Pharmazeutische Zusammensetzung nach Anspruch 1, wobei Rosuvastatin-Calcium in dem Kern in einem Anteil von 2 bis 20%, insbesondere 3,467-13,867% vorhanden ist.

3. Pharmazeutische Zusammensetzung nach Anspruch 1-2, wobei Lactosemonohydrat in dem Kern in einem Anteil von 30-60% vorhanden ist.

4. Pharmazeutische Zusammensetzung nach Anspruch 1-3, wobei microkristalline Cellulose in dem Kern in einem Anteil von 15-35% vorhanden ist.

5. Pharmazeutische Zusammensetzung nach Anspruch 1-4, wobei Crospovidon in dem Kern in einem Anteil von 1-5% vorhanden ist.

6. Pharmazeutische Zusammensetzung nach Anspruch 1-5, wobei Magnesiumstearat in dem Kern in einem Anteil von 0,25-5% vorhanden ist.

7. Verfahren zur Herstellung einer stabilen pharmazeutischen Tablette umfassend Rosuvastatin nach Anspruch 1-6, enthaltend:
a. Pressen der Pulvermischung
b. Beschichten des gepressten Kerns mit einem Film der frei von Eisenoxiden ist, auf eine Gewichtszunahme von 2-10%, insbesondere 3-4%.

8. Pharmazeutische Zusammensetzung nach Ansprüchen 1-7, wobei der Kern 1-40 mg Rosuvastatin, insbesondere 5, 10, 20, 40 mg umfasst.

## Revendications

1. Composition pharmaceutique d'un comprimé obtenu par compression, qui contient de la rosuvastatine calcique amorphe, du carbonate de calcium à titre d'agent stabilisant et du lactose monohydraté à titre de charge soluble, enrobée d'un film à base d'alcool polyvinylique, dépourvu d'oxydes de fer, dans laquelle le carbonate de calcium et le lactose monohydraté sont dans un rapport de 1:3 à 1:14.

2. Composition pharmaceutique selon la revendication 1, dans laquelle la rosuvastatine calcique est présente dans le coeur en une proportion de 2 à 20 %, en particulier de 3,467 à 13,867 %.

3. Composition pharmaceutique selon les revendications 1 et 2, dans laquelle le lactose monohydraté est présent dans le coeur en une proportion de 30 à 60 %.

4. Composition pharmaceutique selon les revendications 1 à 3, dans laquelle une cellulose microcristalline est présente dans le coeur en une proportion de 15 à 35 %.

5. Composition pharmaceutique selon les revendications 1 à 4, dans laquelle une crospovidone est présente dans le coeur en une proportion de 1 à 5 %.

6. Composition pharmaceutique selon les revendications 1 à 5, dans laquelle un stéarate de magnésium est présent dans le coeur en une proportion de 0,25 à 5 %.

7. Procédé de fabrication d'un comprimé pharmaceutique stable comprenant de la rosuvastatine, selon les revendications 1 à 6, comprenant :
a. la compression du mélange pulvérulent
b. l'enrobage du coeur comprimé avec un film dépourvu d'oxydes de fer, jusqu'à un gain de poids de 2 à 10 %, en particulier de 3 à 4 %.

8. Composition pharmaceutique selon les revendications 1 à 7, dans laquelle ledit coeur comprend de 1 à 40 mg de rosuvastatine, en particulier 5, 10, 20, 40 mg.
